# FASCICULE DE BREVET EUROPEEN

(11) **EP 3 838 202 B1**
(45) Date de publication et mention de la délivrance du brevet: **19.06.2024**
(21) Numéro de dépôt: 21156698.9
(22) Date de dépôt: 23.12.2014
(51) Int. Cl.: A61B 17/88, A61B 17/86, A61B 17/70, A61B 17/16, A61B 17/68

(54) **SYSTÈME D'IMPLANT INTRAVERTÉBRAL EXPANSIBLE AVEC FIXATION PÉDICULAIRE POSTÉRIEURE**
EXPANDIERBARES INTRAVERTEBRALES IMPLANTATSYSTEM MIT POSTERIORER PEDIKELFIXIERUNG
SYSTEM FOR EXPANDABLE INTRA-VERTEBRAL IMPLANT WITH REAR PEDICLE ATTACHMENT

(30) Priorité: 23.12.2013 FR 1363467
(43) Date de publication de la demande: 23.06.2021
(62) Demande divisionnaire de: 14831026.1
(73) Titulaire: Stryker European Operations Limited, Carrigtwohill, Co. Cork T45 HX08 (IE)
(72) Inventeur: OGLAZA, Jean-François, 31130 Balma (FR); RATRON, Yves-Alain, 38000 Grenoble (FR); MAESTRETTI, Gianluca, 1784 Wallenreid (CH)
(74) Mandataire: Röthinger, Rainer

(56) Documents cités:
- EP-A1- 2 074 956
- EP-A1- 2 572 680
- US-A1- 2010 324 607
- US-A1- 2012 123 481
- US-B2- 7 291 150

## Description

### DOMAINE DE L'INVENTION

La présente invention concerne un système d'implant intravertébral expansible avec fixation pédiculaire postérieure. Plus particulièrement, la présente invention concerne un système comprenant un implant intravertébral expansible offrant un ancrage vertébral amélioré par l'ajout d'une douille pédiculaire solidement ancrée dans un pédicule vertébral.

### ÉTAT DE LA TECHNIQUE

Plusieurs techniques de vertébroplastie sont connues pour effectuer un redressement vertébral permettant de faire reprendre sa forme ou sa morphologie initiale à une vertèbre déformée suite à une compression osseuse, par exemple due à l'ostéoporose ou à un traumatisme.

On connaît par exemple la technique de kyphoplastie consistant à introduire dans une vertèbre un ballonnet gonflable, puis à envoyer un fluide sous pression dans le ballonnet placé dans la vertèbre afin de forcer l'enveloppe corticale de la vertèbre, et notamment les plateaux vertébraux inférieur et supérieur à reprendre une forme redressée sous l'effet de la pression. Une fois l'enveloppe corticale osseuse redressée, le ballonnet est ensuite dégonflé, puis retiré de la vertèbre, afin de pouvoir injecter dans cette dernière un ciment osseux destiné à conférer à la vertèbre redressée une résistance mécanique stable dans le temps.

On connaît également, par l'intermédiaire notamment de la demande de brevet internationale WO2005/120400, un implant expansible comprenant un premier et un deuxième plateaux opposés, aptes à former respectivement une première et une deuxième surfaces d'appui dans un corps vertébral ; ces deux surfaces d'appui étant appelées à s'éloigner l'une dans l'autre suivant un plan d'expansion prédéfini. L'implant expansible est positionné dans le corps vertébral, et les plateaux sont déployés suivant un plan d'expansion qui correspond au plan de redressement osseux souhaité. Un ciment osseux est ensuite injecté afin de stabiliser le redressement osseux. Le ciment osseux pourra être injecté avec une pression relativement faible grâce à l'implant qui reste en place dans le corps vertébral.

Dans les cas des traumatismes les plus sévères, l'emploi des méthodes précitées peut se révéler insuffisant. La réparation des fractures vertébrales, et notamment des fractures vertébrales par compression s'effectue alors préférentiellement par le biais de vis insérées dans les pédicules des vertèbres sus et sous-jacentes à la vertèbre compressée, ces vis étant associés à des barres postérieures, reliant ainsi mécaniquement les deux vertèbres sus et sous-jacente pour permettre une consolidation vertébrale. Les vis pédiculaires sont bien connues dans l'état de la technique. On connaît notamment des vis tels que décrites dans le brevet US 5,209,753.

L'inconvénient de cette technique réside dans la fusion de deux niveaux articulaires vertébraux engendrée par la fixation des barres postérieures reliant les vertèbres sus et sous-jacentes. De plus ces méthodes ne permettent pas toujours le redressement osseux de la vertèbre compressée mais uniquement la stabilisation de trois vertèbres adjacentes et donc le blocage de deux niveaux articulaires. Le geste chirurgical associé à cette procédure est par ailleurs très invasif et oblige à accéder à au moins deux vertèbres.

Afin de pallier à ces inconvénients, il apparaît nécessaire de fournir un dispositif permettant la réparation de fractures vertébrales, et notamment de fractures vertébrales sévères par compression, évitant la fusion et permettant la consolidation, au niveau du pédicule, d'un implant intravertébral expansible situé dans le corps vertébral.

Ainsi, l'invention a pour objet d'associer à un implant intravertébral expansible, positionné dans un corps vertébral, une fixation (par exemple douille ou chemise) pédiculaire, destinée à être ancrée dans le pédicule. Cette fixation pédiculaire assure un appui et un ancrage osseux de grande qualité. En effet, si le corps vertébral est constitué d'os spongieux, ayant une porosité importante de 30 à 90%, le pédicule est constitué d'os cortical, ayant une porosité de 5 à 30%, offrant ainsi un appui mécanique solide pour l'implant intravertébral et permettant de reconstruire les fractures vertébrales, et notamment les fractures vertébrales par compression, même les plus sévères.

Des systèmes d'implants osseux extensibles comprenant un ancrage supplémentaire dans l'os pédiculaire sont connus, comme par exemple les systèmes décrits dans les demandes de brevet EP 2 074 956 et US 2009/005821. Cependant, ces systèmes décrivent un membre expansible rigidement connecté à ladite fixation pédiculaire, par exemple par vissage des deux parties. De ce fait, la direction d'expansion du membre expansible ainsi que le positionnement du membre au sein du corps vertébral sont directement dépendants de la position de la fixation pédiculaire.

L'invention présente l'avantage de permettre le déploiement de l'implant intravertébral expansible indépendamment, selon au moins un degré de liberté, de la position de la fixation pédiculaire. Le système d'implant permet aussi de solidariser (i.e. de bloquer tous les degrés de liberté) ledit implant intravertébral extensible par rapport à la fixation pédiculaire, lorsque le système d'implant est positionné en extension dans la vertèbre.

Avantageusement, on associe à l'élément de fixation pédiculaire un élément postérieur servant de système de connexion, permettant d'adjoindre à l'implant intravertébral expansible des moyens complétant le redressement osseux, par exemple par des barres postérieures ou d'autres systèmes visant à stabiliser encore davantage le foyer de fracture.

Ainsi la présente invention assure un redressement vertébral à l'aide d'un système d'implant intravertébral expansible modulable avec fixation pédiculaire postérieure.

### RÉSUMÉ

L'invention concerne donc un système d'implant intravertébral expansible comprenant un implant intravertébral comprenant une partie antérieure expansible dans un corps vertébral et une partie postérieure; et une fixation pédiculaire ayant au moins une portion creuse de réception de la partie postérieure de l'implant intravertébral.

Selon un mode de réalisation, la fixation pédiculaire comprend un filetage assurant l'ancrage de ladite fixation pédiculaire dans un pédicule vertébral.

Selon un mode de réalisation, la fixation pédiculaire comprend une portion interne postérieure creuse et une portion interne antérieure creuse dans laquelle la partie postérieure de l'implant intravertébral peut se déplacer selon au moins un degré de liberté.

Selon un mode de réalisation, la fixation pédiculaire comprend un axe principal et la partie postérieure de l'implant intravertébral peut se déplacer en translation et en rotation, selon l'axe principal de la fixation pédiculaire, dans la portion interne antérieure de la fixation pédiculaire ; de manière à ce que la direction d'expansion de la partie antérieure de l'implant intravertébral soit indépendante de la position de la fixation pédiculaire dans le pédicule vertébral.

Selon un mode de réalisation, la partie antérieure de l'implant intravertébral comprend un premier et un deuxième plateaux, aptes à former respectivement une première et une deuxième surfaces d'appui dans un corps vertébral ; ces deux surfaces pouvant s'éloigner l'une dans l'autre suivant un plan d'expansion prédéfini.

Selon un mode de réalisation, la portion interne antérieure comprend au moins une rainure sur sa surface interne.

Selon un mode de réalisation, une portion postérieure de la partie postérieure de l'implant intravertébral comprend un évidement cylindrique et au moins un moyen de solidarisation permettant de bloquer en rotation et en translation les mouvements relatifs entre l'implant intervertébral et la fixation pédiculaire.

Selon un mode de réalisation, le moyen de solidarisation comprend au moins un orifice traversant, traversant la portion postérieure et une chambre périphérique à la surface de la portion postérieure en communication fluide avec le au moins un orifice traversant.

Selon un mode de réalisation, le moyen de solidarisation comprend au moins deux fentes s'étendant axialement le long de la portion postérieure, un taraudage interne et une extrémité postérieure évasée; pouvant coopérer avec un bouchon conique d'expansion.

Selon un mode de réalisation, l'élément postérieur est solidaire de la fixation pédiculaire par l'intermédiaire d'une tige filetée vissée dans un taraudage de la portion interne postérieure.

Selon un mode de réalisation, l'élément postérieur comprend une portion postérieure permettant l'assemblage avec des éléments de fixation postérieurs complémentaires tels que des barres ou des ligaments artificiels.

### BRÈVE DESCRIPTION DES FIGURES

D'autres particularités et avantages ressortiront clairement de la description qui en est faite ci-après, à titre indicatif et nullement limitatif, en référence aux dessins annexés, dans lesquels :
Figure 1 est une vue éclatée du système d'implant selon un mode de réalisation de la présente invention.
Figure 2 est une vue latérale de l'implant intravertébral selon un mode de réalisation de la présente invention.
Figure 3A est une vue latérale de la fixation pédiculaire selon un mode de réalisation de la présente invention. Figure 3B est une vue en coupe de la fixation pédiculaire selon un mode de réalisation de la présente invention.
Figure 4 est une vue en perspective de l'élément postérieur selon un mode de réalisation de la présente invention.
Figure 5 est une vue latérale du système d'implant selon un mode de réalisation de la présente invention, monté sur un instrument adapté pour l'insertion du système d'implant.
Figure 6A est une vue latérale du système d'implant, selon un mode de réalisation de la présente invention, placé dans une vertèbre ; ladite vertèbre étant représentée transparente, visible uniquement par ses contours.
Figure 6B est une vue de dessus du système d'implant, selon un mode de réalisation de la présente invention, placé dans une vertèbre ; ladite vertèbre étant représentée transparente, visible uniquement par ses contours.
Figure 7A est une vue en coupe du système d'implant selon un mode de réalisation de la présente invention, avant expansion de la partie antérieure de l'implant.
Figure 7B est une vue en coupe du système d'implant selon un mode de réalisation de la présente invention, après expansion de la partie antérieure de l'implant.
Figure 8A est une vue en coupe du système d'implant, après expansion de la partie antérieure de l'implant illustrant les moyens de solidarisation de la fixation pédiculaire à l'implant intravertébral selon un mode de réalisation de la présente invention.
Figure 8B est une vue en perspective de l'implant intervertébral, après expansion de la partie antérieure de l'implant illustrant les moyens de solidarisation de la fixation pédiculaire à l'implant selon un mode de réalisation de la présente invention.
Figure 8C est une vue en perspective de la fixation pédiculaire illustrant notamment la portion interne antérieure selon un mode de réalisation de la présente invention.
Figure 9A est une vue en coupe du système d'implant, après expansion de la partie antérieure de l'implant illustrant les moyens de solidarisation de la fixation pédiculaire à l'implant intravertébral selon un mode de réalisation de la présente invention.
Figure 9B est une vue en perspective de l'implant, après expansion de la partie antérieure de illustrant les moyens de solidarisation de la fixation pédiculaire à l'implant intravertébral selon un mode de réalisation de la présente invention.
Figure 10A est une vue latérale du système d'implant selon un mode de réalisation de la présente invention placé dans une vertèbre ; ladite vertèbre étant représentée transparente, visible uniquement par ses contours.
Figure 10B est une vue en coupe du système d'implant selon un mode de réalisation de la présente invention placé dans une vertèbre ; ladite vertèbre étant représentée transparente, visible uniquement par ses contours.

Les dessins des figures ne sont pas à l'échelle. Il va de soi que la portée de l'invention ne se limite pas aux exemples de réalisation plus spécialement décrit et représentés en référence aux dessins annexés ; elle en embrasse au contraire toutes les variantes.

### RÉFÉRENCES

1 Implant intravertébral,
11 Partie antérieure de l'implant intravertébral,
12 Partie postérieure de l'implant intravertébral,
121 Épaulement,
122 Évidement cylindrique,
123 Chambre,
124 Orifice traversant,
125 Fente,
126 Taraudage interne,
127 Extrémité postérieure évasée,
128 Portion postérieure de la partie postérieure de l'implant intravertébral,
13 Tube de traction central,
131 Orifice traversant,
2 Fixation pédiculaire, notamment douille pédiculaire,
21 Filetage,
22 Portion interne postérieure,
221 Taraudage,
23 Portion interne antérieure,
231 Rainure,
24 Moyen d'entraînement en rotation de la fixation pédiculaire - Encoche,
25 Chanfrein d'entrée,
3 Élément postérieur, 31 Portion antérieure,
311 Filetage,
32 Portion postérieure,
33 Surface d'appui,
4 Vertèbre,
5 Instrument d'insertion,
51 Canule solidaire en rotation de la fixation pédiculaire,
52 Tube d'expansion,
6 Bouchon conique d'expansion.

### DESCRIPTION DÉTAILLÉE

La présente invention concerne un système d'implant intravertébral expansible avec fixation pédiculaire postérieure, comme présenté dans la revendication 1. Les modes de réalisation préférés sont décrits dans les revendications dépendantes.

Selon un premier mode de réalisation, le système d'implant comprend un implant intravertébral associé à une fixation pédiculaire assurant un ancrage supplémentaire au niveau du pédicule vertébral.

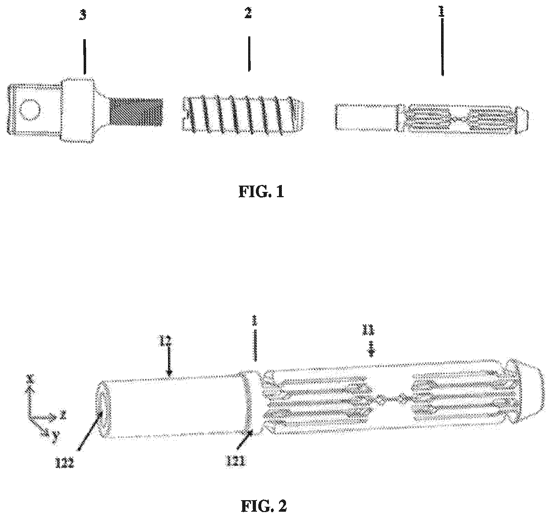

Selon un second mode de réalisation, comme représenté sur la Figure 1, le système d'implant comprend un implant intravertébral 1 associé à une fixation pédiculaire 2 et à un élément postérieur 3 permettant de connecter, postérieurement à la vertèbre, tout dispositif visant à stabiliser, renforcer ou réparer la fracture vertébrale, notamment la fracture vertébrale par compression.

### Implant intravertébral

Comme représenté sur la Figure 2, l'implant intravertébral 1 selon la présente invention comprend une partie antérieure 11 et une partie postérieure 12.

Selon un mode de réalisation, la partie antérieure 11 correspond à un implant intravertébral 1 déformable et expansible tel que l'implant SpineJack^{®} commercialisé par la société VEXIM, connu de l'homme du métier et également décrit dans la demande de brevet EP 1 778 136, incorporée ici par référence. Il est précisé que l'homme du métier, à la lecture de ladite demande de brevet saurait facilement retrouver les caractéristiques nécessaires pour la réalisation de la présente invention. En particulier, la partie antérieure 11 de l'implant intravertébral 1 de la présente invention consiste en une partie expansible permettant un redressement osseux et comprenant :
- un plan d'expansion unique déterminé, intrinsèque à ladite partie antérieure 11,
- un premier et un deuxième plateaux opposés, aptes à former respectivement une première et une deuxième surfaces d'appui dans un corps vertébral destinées à s'éloigner l'une de l'autre suivant ledit plan d'expansion unique lors de l'expansion de l'implant,
- un premier et un deuxième appuis pour chacun desdits premier et deuxième plateaux, situés sous ces derniers respectivement, et
- des moyens de réglage d'une valeur déterminée d'expansion, comportant un voile de matière disposé entre chaque appui et un plateau correspondant, ledit voile possédant une épaisseur déterminée qui permet de contrôler l'expansion de l'implant. Selon un mode de réalisation, ladite partie antérieure 11 comprend également un tube de traction central 13 permettant de piloter l'expansion des premiers et deuxièmes plateaux. Selon un mode de réalisation alternatif, la partie antérieure 11 de l'implant intravertébral 1 correspond à tout implant intravertébral connu de l'homme du métier qui soit déformable, expansible et apte à rester dans le corps vertébral après expansion.

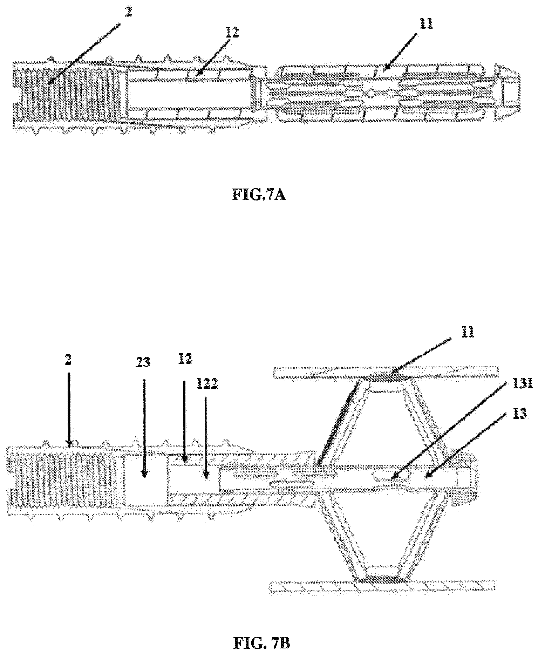

Selon un mode de réalisation, tel que représenté sur les Figures 7A-B, 8A-B, 9A-B et 10A-B, le tube de traction central 13 est creux et comprend au moins un orifice traversant 131 permettant l'injection d'un matériau de remplissage dans la vertèbre 4, tel que du ciment osseux, après expansion des premier et deuxième plateaux. Selon un mode de réalisation, le tube de traction central 13 comprend au moins 1, 2, 3, 4, 5, 6, 7, 8, 9, 10 orifices traversant 131. Selon un mode de réalisation, le(s) orifice(s) traversant 131 traverse radialement le tube de traction central 13 afin de permettre une communication fluide entre le conduit interne et la surface externe. Selon un mode de réalisation, le tube de traction central 13 comprend une pluralité d'orifices traversant 131 régulièrement répartis sur sa surface. Selon un mode de réalisation, le au moins un orifice traversant 131 se situe entre les deux plateaux afin d'être en communication fluide avec l'intérieur de la vertèbre après l'expansion de l'implant et l'écartement des deux plateaux. Avantageusement, l'injection d'un matériau de remplissage dans la vertèbre 4 permet de maintenir la partie antérieure 11 de l'implant 1 en position dans le corps vertébral après expansion. Selon un mode de réalisation, le matériau de remplissage est un ciment osseux biocompatible, par exemple un ciment ionique, un ciment phosphocalcique, un ciment acrylique ou un composé de ceux-ci, ou une résine. Selon un mode de réalisation, le matériau de remplissage se solidifie après injection. Avantageusement, la solidification du matériau de remplissage permet de consolider la vertèbre 4 et de renforcer la solidité de l'implantation du système d'implant dans la vertèbre 4.

Selon un mode de réalisation, la partie postérieure 12 est un corps cylindrique creux relié, à son extrémité antérieure, à l'extrémité postérieure de la partie antérieure 11 par un épaulement 121. Ladite partie postérieure 12 comprend un évidement cylindrique 122 au travers duquel peut coulisser un tube de traction central 13, situé initialement dans la partie antérieure 11. Par traction sur le tube de traction central 13, le tube de traction central 13 coulisse dans l'évidement cylindrique 122 de la partie postérieure 12 et les extrémités antérieures et postérieures de la partie antérieure 11 se rapproche entraînant l'expansion des premiers et deuxièmes plateaux de la partie antérieure 11 de l'implant intravertébral 1. Le tube de traction central 13 permet de piloter le déploiement de la partie antérieure déformable expansible 11. Une fois le tube de traction 13 tracté à l'intérieur de l'évidement 122, le tube ne peut plus revenir à sa position initiale, ce qui maintient l'expansion de la partie antérieure 11, en attendant l'injection de ciment ou substitut osseux.

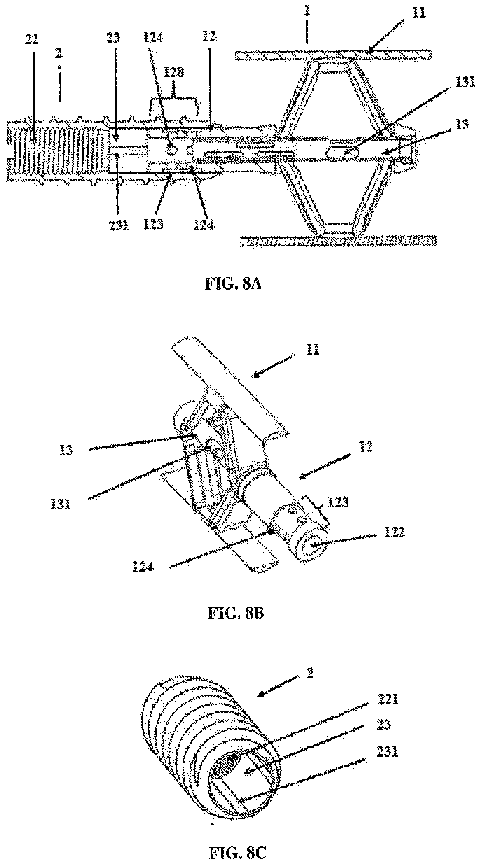

Selon un mode de réalisation illustré notamment par la Figure 8B, la partie postérieure 12 de l'implant intravertébral 1 comprend un évidement cylindrique 122 et au moins un orifice traversant 124. Avantageusement le au moins un orifice traversant se situe dans la portion postérieure 128 de la partie postérieure 12. Selon un mode de réalisation, la portion postérieure 128 correspond à la portion de la partie postérieure de l'implant intravertébral 12 qui reste en permanence à l'intérieur de la portion interne antérieure 23. Selon un mode de réalisation, l'évidement cylindrique 122 traverse toute la partie postérieure de l'implant 12. Selon un mode de réalisation, la partie postérieure de l'implant intravertébral 12, et notamment la portion postérieure 128 comprend en outre une portion avec un diamètre externe réduit définissant ainsi une chambre périphérique 123. Selon un mode de réalisation, le au moins un orifice traversant 124 traverse la partie postérieure de l'implant 12 depuis l'évidement cylindrique 122 jusqu'à la chambre périphérique 123. Selon un mode de réalisation, la partie postérieure 12 de l'implant, et notamment sa portion postérieure 128 comprend au moins 1, 2, 3, 4, 5, 6, 7, 8, 9, 10 orifices traversant 124. Selon un mode de réalisation, les orifices traversant 124 sont régulièrement répartis sur la portion postérieure 128.

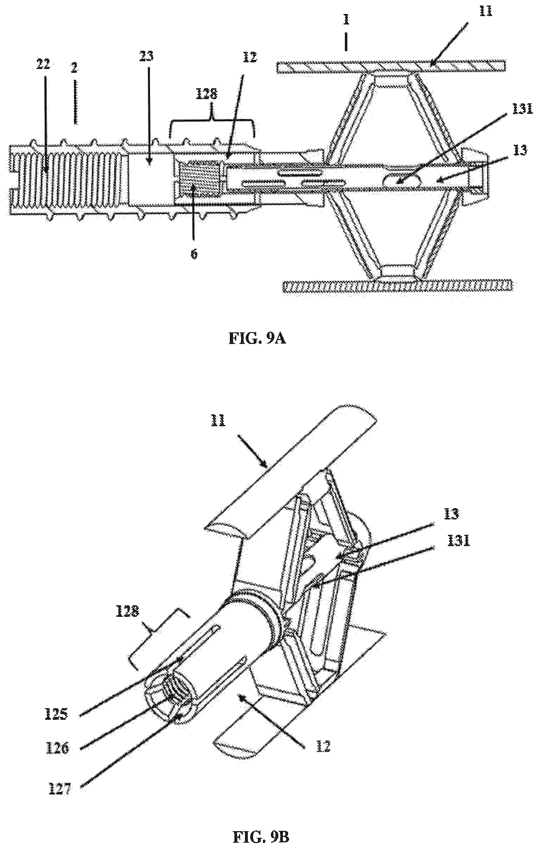

Selon un mode de réalisation illustré par la Figure 9B, la partie postérieure de l'implant intravertébral 12 comprend un évidement cylindrique 122 et au moins une fente 125 traversant la partie postérieure 12 et s'étendant axialement. Avantageusement la au moins une fente 125 se situe dans la portion postérieure 128 de la partie postérieure 12. Selon un mode de réalisation, la portion postérieure 128 correspond à la portion de la partie postérieure de l'implant intravertébral 12 qui reste en permanence à l'intérieur de la portion interne antérieure 23. Selon un mode de réalisation, l'évidement cylindrique 122 traverse toute la partie postérieure de l'implant 12. Selon un mode de réalisation, la partie postérieure de l'implant intravertébral 12, et notamment la portion postérieure 128 comprend en outre un taraudage interne 126 situé sur la surface interne de l'évidement cylindrique 122. Selon un mode de réalisation, la partie postérieure de l'implant intravertébral 12, et notamment la portion postérieure 128 comprend en outre une extrémité postérieure évasée 127 pouvant coopérer avec un bouchon conique d'expansion 6. Selon un mode de réalisation, la partie postérieure 12 comprend au moins 1, 2, 3, 4, 5, 6, 7, 8, 9, 10 fentes 125. Selon un mode de réalisation, la fente 125 s'étend sur tout ou partie de la portion postérieure 128. Selon un mode de réalisation, le taraudage 126 s'étend sur tout ou partie de la portion postérieure 128.

Selon un mode de réalisation, l'expansion de l'implant intervertébral 1 n'est pas due à l'injection d'un produit dans la partie postérieure de l'implant 1. Cependant, l'injection d'un matériau de remplissage peut permettre de stabiliser l'implant une fois dans sa position en expansion. Selon un mode de réalisation, l'implant intervertébral 1 ne comprend pas une poche destinée à être emplie d'un produit (e.g. un matériau de remplissage) afin de permettre l'expansion de la poche. Selon un mode de réalisation, la seule injection d'un matériau de remplissage au travers de la partie antérieure 11 ne permet pas l'expansion de l'implant intervertébral 1.

Selon un mode de réalisation, la partie postérieure 12 de l'implant intervertébral 1 ne comprend pas de filetage externe destiné à être vissé avec la fixation pédiculaire 2.

### Fixation pédiculaire

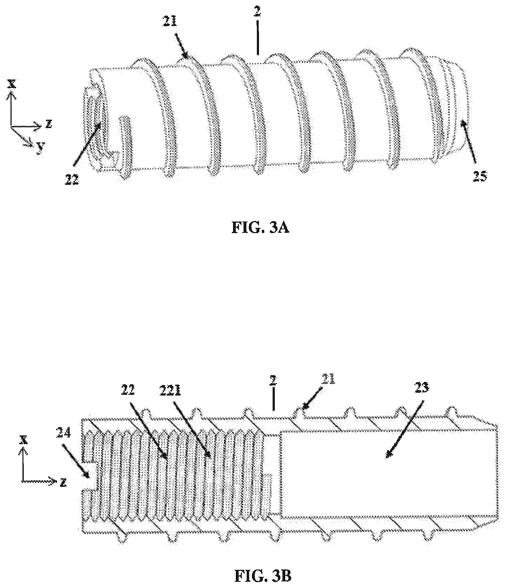

Comme représentée sur la Figure 3A, la fixation pédiculaire 2 selon la présente invention comprend au moins un filetage 21. Selon un mode de réalisation, la fixation pédiculaire 2 comprend un filetage 21 sur la surface externe de la fixation pédiculaire 2. Ledit filetage 21 externe est apte à fournir un ancrage osseux dans le pédicule. L'ancrage de la fixation pédiculaire 2 dans un pédicule vertébral offre un appui mécanique supplémentaire à l'implant intravertébral 1, et notamment à la partie antérieure expansible 11 dudit implant 1. L'insertion de la fixation pédiculaire 2 dans le tissu osseux est facilitée par un chanfrein 25 situé à l'extrémité antérieure de la fixation pédiculaire 2. Selon un mode de réalisation, la surface externe de la fixation pédiculaire 2 ne présente pas de texture autre que celle induite par un filetage, comme par exemple une structure maillée, striée ou mouchetée. Comme représentée sur la Figure 3B, la fixation pédiculaire 2 est une douille ou chemise pédiculaire cylindrique creuse comprenant une portion interne postérieure 22 et une portion interne antérieure 23. Selon un mode de réalisation, la fixation pédiculaire 2 n'est pas une vis pédiculaire pleine comprenant une portion partielle interne creuse. Selon un mode de réalisation, la fixation pédiculaire 2 est creuse sur toute sa longueur. Selon un mode de réalisation, les portions internes postérieure 22 et antérieure 23 ne présentent pas le même état de surface.

Selon un mode de réalisation, la portion interne postérieure 22 comprend un taraudage 221 ou tout autre moyen à la portée de l'homme du métier permettant de relier la fixation 2 à un élément postérieur 3.

Selon un mode de réalisation, la portion interne antérieure 23 comprend un alésage définissant une portion creuse de réception de la partie postérieure 12 de l'implant intravertébral 1. Selon un mode de réalisation, la surface de la portion interne antérieure 23 est lisse. Selon un mode de réalisation, la surface de la portion interne antérieure 23 n'est pas taraudée. Selon un mode de réalisation, l'implant intravertébral 1, et notamment la partie postérieure 12, ne traverse pas l'intégralité de la fixation pédiculaire creuse 2. Selon un mode de réalisation, la partie postérieure 12 de l'implant intervertébral 1 est destinée à être insérée à l'intérieur de la portion interne antérieure 23. Selon un mode de réalisation, la partie postérieure 12 sert de guidage à la fixation pédiculaire 2. L'épaulement 121 sert ainsi de butée de guidage pour la fixation 2. Selon un mode de réalisation, la partie postérieure 12 de l'implant intravertébral 1 peut se déplacer selon au moins un degré de liberté, préférentiellement 2 degrés de liberté dans la portion interne antérieure 23 de la fixation pédiculaire 2. Selon un mode de réalisation, la partie postérieure 12 de l'implant intervertébral 1 est coulissante dans la portion interne antérieure 23. Selon un mode de réalisation, la liaison mécanique entre la partie postérieure 12 et la portion interne antérieure 23 est une liaison pivot glissant. Selon un mode de réalisation, la fixation pédiculaire 2, notamment la douille pédiculaire, définit un axe principal. Selon ce mode de réalisation, la partie postérieure 12 de l'implant intravertébral 1 peut se déplacer en rotation et en translation selon ledit axe principal dans la portion interne antérieure 23 de la fixation pédiculaire 2.

Avantageusement, la portion antérieure 23 permet l'expansion de l'implant intervertébral 1 indépendamment de l'ancrage de la fixation pédiculaire 2 dans le pédicule vertébral. En effet, le degré de liberté en translation selon l'axe principal de la fixation pédiculaire 2 permet de ne pas contraindre le positionnement (i.e. l'avancement dû au déploiement) de l'implant intervertébral 1 dans le corps vertébral. De plus, le degré de liberté en rotation autour de l'axe principal de la fixation pédiculaire 2 permet de ne pas contraindre la direction d'expansion de l'implant intervertébral 1. Ainsi, l'implant intervertébral 1, lorsqu'il est déployé, adapte sa position de déploiement en fonction du milieu dans lequel il est déployé (i.e. le corps vertébral).

Selon un mode de réalisation, la portion interne antérieure 23 ne comprend pas de filetage. Selon un mode de réalisation, la portion interne antérieure 23 et l'implant intervertébral 1 ne sont pas vissés.

Selon un mode de réalisation, la portion interne antérieure 23 comprend en outre au moins une rainure 231, comme représenté sur la Figure 8C. Selon un mode de réalisation, la portion interne antérieure 23 comprend au moins une rainure axiale (i. une rainure parallèle à l'axe z). Selon un mode de réalisation, la portion interne antérieure 23 comprend au moins une rainure transverse (i.e. perpendiculaire à l'axe z). Selon un mode de réalisation, la portion interne antérieure 23 comprend au moins une rainure oblique 231. Selon un mode de réalisation, la portion interne antérieure 23 comprend au moins une rainure 231 axiale et/ou transverse et/ou oblique. Selon un mode de réalisation, la au moins une rainure 231 comprend une section non constante. Selon un mode de réalisation, la portion interne antérieure 23 comprend au moins 2, 3, 4, 5, 6, 7, 8, 9, 10 rainures 231. Selon un mode de réalisation, les rainures sont régulièrement réparties sur la surface interne de la portion antérieure 12. Selon un mode de réalisation, la fixation pédiculaire 2 comprend au moins une encoche 24, ou tout autre moyen à la portée de l'homme du métier, permettant d'entraîner en rotation la fixation pédiculaire 2, à l'aide d'un instrument d'insertion 5.

### Élément postérieur

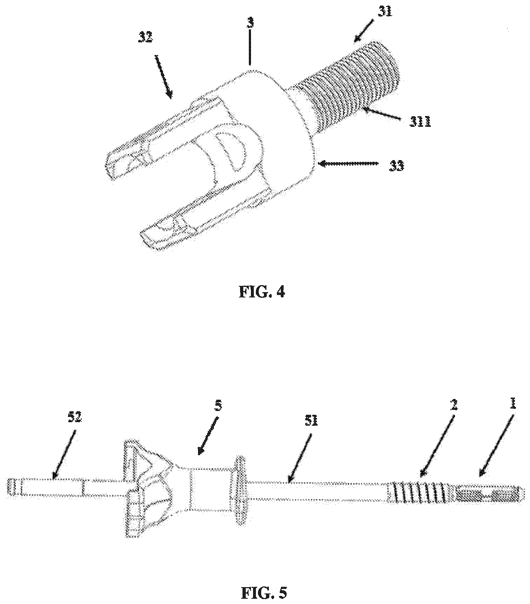

Comme représenté sur la Figure 4, l'élément postérieur 3 selon la présente invention comprend une portion antérieure 31 constituée d'une tige, de préférence une tige filetée 311 coopérant avec le taraudage 221 de la fixation pédiculaire 2, permettant ainsi la fixation de l'élément postérieur 3 à la fixation pédiculaire 2. L'élément postérieur 3 comprend également une portion postérieure 32, externe à la vertèbre 4, dont la géométrie permet d'assurer des fonctions d'assemblage avec des éléments de fixation postérieurs complémentaires et connus de l'homme du métier comme des barres ou des ligaments artificiels. Toute autre configuration d'assemblage à d'autres systèmes connu de l'homme du métier peut être envisagée. L'élément postérieur 3 comprend également une surface d'appui 33 contre le tissu osseux ou contre la portion postérieure de la fixation pédiculaire 2, selon le choix effectué par l'opérateur et/ou l'enfoncement de la fixation pédiculaire 2 dans l'os pédiculaire.

### Fonctionnement

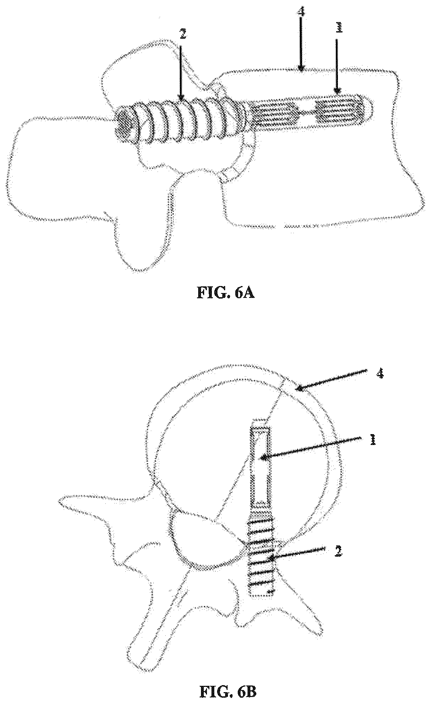

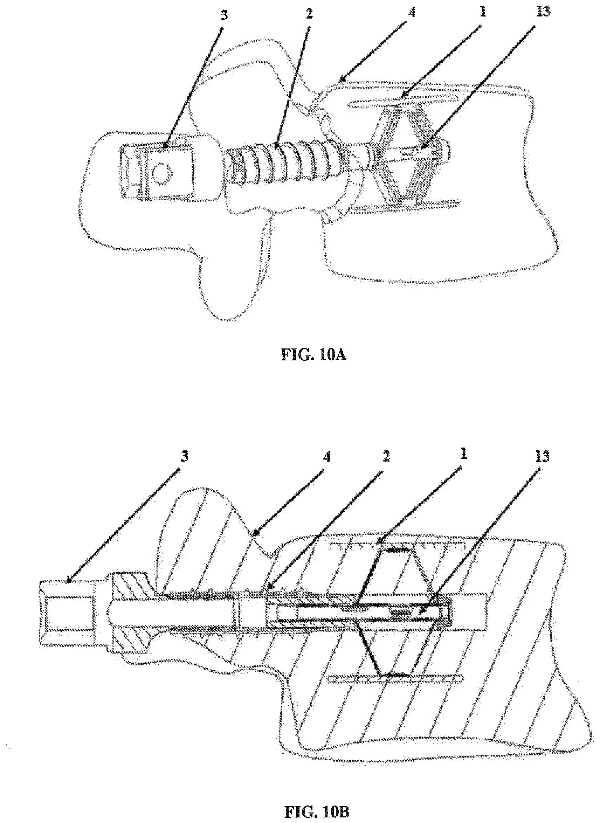

Après la création d'une voie d'abord et l'alésage du pédicule vertébral, le système d'implant comprenant la fixation pédiculaire 2 et l'implant vertébral 1 dont la partie postérieure 12 est insérée dans la portion interne antérieure 23 de la fixation pédiculaire 2 est inséré, puis vissé, dans le pédicule l'aide d'un instrument d'insertion 5, comme représenté sur la Figure 5. Ledit instrument d'insertion 5 comprend une canule de travail 51 solidaire (i.e. connectée) en rotation de la fixation pédiculaire 2 par l'intermédiaire des encoches 24 permettant de visser la fixation pédiculaire 2 dans le pédicule. Ledit instrument d'insertion 5 comprend également un tube d'expansion 52 situé le long de l'axe principal de l'instrument d'insertion 5 et il est configuré pour traverser au moins la longueur de la portion interne postérieure 22. Le tube d'expansion 52 est solidaire (i.e. connecté) de la partie postérieure 12 de l'implant intravertébral 1, et notamment solidaire du tube de traction central 13 de l'implant intravertébral 1. La profondeur d'insertion du système d'implant est contrôlée par l'opérateur par le vissage du filetage 21 de la fixation pédiculaire 2 dans le tissu osseux, afin de positionner l'implant intravertébral 1 de manière optimale dans le corps vertébral. Le positionnement de l'implant intravertébral 1 en rotation axiale est contrôlé par l'opérateur de manière indépendante de l'insertion de la fixation pédiculaire 2, à l'aide du tube 52 de manière à conserver un plan d'expansion, par exemple un plan d'expansion cranio-caudal de l'implant intravertébral 1 quel que soit l'angle de vissage obtenu lors de l'insertion de la fixation pédiculaire 2. Comme représenté sur les Figures 6A, 6B, 10A et 10B, le système d'implant intravertébral selon la présente invention est inséré à travers un pédicule vertébral, l'implant intravertébral 1 étant alors situé dans le corps vertébral et la fixation pédiculaire 2 étant ancrée dans le pédicule.

Selon un mode de réalisation, après insertion du système d'implant, la fixation pédiculaire 2 est solidement fixée dans le pédicule et l'implant intravertébral 1 est partiellement solidaire de la fixation pédiculaire 2 par sa partie postérieure cylindrique 12. En effet, la partie postérieure cylindrique 12 est insérée dans la portion interne antérieure 23 bloquant ainsi les mouvements de rotations autour des axes y et x et bloquant les translations le long desdits axes x et y. De ce fait, ces degrés de liberté bloqués assurent une solidarité partielle entre la partie postérieure cylindrique 12 insérée dans la portion interne antérieure 23. Par solidarisation on entend le blocage de tous les degrés de liberté entre la fixation pédiculaire 2 et l'implant intravertébral 1. Par solidarisation partielle, on entend, le blocage d'au moins un degré de liberté entre ces deux parties. La fixation pédiculaire 2 vient alors en butée sur l'épaulement 121. Ainsi l'implant intravertébral 1 possède un point d'appui supplémentaire dans le pédicule. Selon un mode de réalisation, l'implant vertébral 1 n'est pas solidaire de la fixation pédiculaire 2 par vissage. Selon un mode de réalisation, la partie postérieure 12 de l'implant intervertébral 1 assemblée à la fixation pédiculaire 2 ne traverse pas de part en part la fixation pédiculaire 2. Selon un mode de réalisation, la partie postérieure 12 de l'implant vertébral 1 est montée dans la portion interne antérieure 23 de la fixation pédiculaire 2.

Comme représentée sur les Figures 7A et 7B, l'expansion de l'implant intravertébral 1 est réalisée par traction sur le tube de traction central 13, à travers le tube 52 de l'instrument d'insertion 5 (non représenté sur les figures). Le tube de traction central 13 peut coulisser unidirectionnellement dans la partie postérieure 12 de l'implant 1 afin de permettre le déploiement tout en empêchant le retrait. La traction du tube de traction central 13 permet le déploiement de l'implant intervertébral 1 par rapprochement des extrémités antérieure et postérieure de la partie antérieure 11. Selon un mode de réalisation, le déploiement permet l'écartement des plateaux dans le corps vertébral. Cette expansion dans le corps vertébral provoque le déplacement antérieur de l'implant : la partie antérieure 11 de l'implant expansible coulisse au travers de la portion interne antérieure 23 afin que la partie antérieure 11 de l'implant intervertébrale s'adapte de manière optimale à l'environnement dans lequel elle se déploie. Avantageusement, la liaison coulissante entre la portion interne postérieure 23 de la fixation pédiculaire 2 et de la partie postérieure 12 de l'implant 1 permet l'adaptation de la position de l'implant intervertébral 1 déployé dans le corps vertébral indépendamment de la position de la fixation pédiculaire 2.

Comme représentée sur la Figure 7B, la partie postérieure 12 peut coulisser librement dans la portion interne antérieure 23, sans toutefois en sortir afin d'assurer un appui supplémentaire permanent, de manière à ne pas contraindre le déploiement de l'implant intravertébral dans une position dépendante de la localisation de la fixation pédiculaire 2 dans le pédicule. En effet, la portion interne antérieure 23 est configurée pour bloquer les translations et les rotations de l'implant intervertébral 1 selon les axes x et y de la fixation pédiculaire 2, et permettre la translation et la rotation selon l'axe principal de la fixation pédiculaire 2 (i.e. l'axe z).

Selon un mode de réalisation, les dimensions de la partie postérieure 12 de l'implant 1 sont telles qu'une fois la partie postérieure 12 insérée dans la portion interne antérieure 23, l'implant 1 ne peut plus être extrait de la fixation pédiculaire lorsqu'il est implanté. La dimension longitudinale de la portion interne antérieure 23 est suffisamment longue pour que la partie postérieure 12 reste insérée et partiellement solidaire de ladite portion interne antérieure 23 lors du déploiement. Selon un mode de réalisation, la longueur de la portion interne antérieure 23 est en effet comprise entre 1 et 20 mm ou entre 5 et 15 mm.

Selon ce mode de réalisation, l'implant intravertébral 1 est libre en rotation axiale et en translation antérieure par rapport à la fixation pédiculaire 2. Le déploiement de l'implant intravertébral 1 se produit ainsi uniquement en fonction de l'environnement osseux intravertébral qui impose à la partie antérieure 11 extensible de l'implant intervertébrale 1 l'orientation et la profondeur de déploiement de ladite partie extensible. Selon un mode de réalisation, l'implant intravertébral, et notamment sa partie postérieure 12, peut se déplacer selon un, deux, trois, quatre, cinq degrés de liberté, préférentiellement deux degrés de liberté, encore plus préférentiellement un degré de liberté en rotation et un degré de liberté en translation, dans la portion interne antérieure 23. Selon un mode de réalisation, l'implant intravertébral 1, et notamment sa partie postérieure 12, peut se déplacer en rotation et en translation selon l'axe z dans la portion interne antérieure 23.

Selon un mode de réalisation, après l'expansion de l'implant intravertébral 1, la rotation et la translation suivant z entre l'implant intravertébral et la fixation pédiculaire sont bloquées, rendant ainsi solidaire l'implant intravertébral expansible 1 de la fixation pédiculaire 2.

Selon un mode de réalisation, la partie antérieure de l'implant intravertébral 11 est stabilisée en position d'expansion par injection d'un matériau de remplissage dans la vertèbre 4, au travers du au moins un orifice traversant 131. Le matériau de remplissage est injecté au moyen d'un injecteur au travers de la fixation pédiculaire 2, de l'évidement cylindrique 122 de la partie postérieure de l'implant 12 et du tube de traction creux 13, jusqu'au niveau de la partie antérieure 11 de l'implant 1.

Selon un mode de réalisation, la partie postérieure de l'implant intravertébral 12 est solidarisée à la fixation pédiculaire 2 en bloquant les mouvements relatifs de la portion postérieure 12 par rapport à la fixation pédiculaire 2 à l'aide de moyens de solidarisation.

Selon un mode de réalisation, tel qu'illustré sur la Figure 8 A, la partie postérieure de l'implant intravertébral 12 est solidarisée dans la fixation pédiculaire 2 par injection d'un matériau de remplissage. Le matériau de remplissage est injecté au travers de la portion interne postérieure 22 dans l'évidement cylindrique 122 et dans le au moins un orifice traversant 124 afin d'entrer en contact avec la surface interne de la portion interne antérieur 23. Avantageusement la portion postérieure 128 comprend une chambre périphérique 123 dans laquelle débouche le au moins un orifice traversant 124. Ainsi le matériau de remplissage rempli le au moins un orifice traversant 124 et la chambre périphérique 123 afin de répartir le matériau de remplissage à l'interface entre la portion postérieure 128 et la portion interne antérieure 23 et ainsi augmenter la surface de contact. Afin de renforcer encore la solidarisation, la portion interne antérieure 23 comprend au moins une rainure 231 et le matériau de remplissage injecté dans l'évidement cylindrique 122 rempli le au moins un orifice traversant 124, la chambre périphérique 123 et la au moins une rainure 231 de la portion interne antérieure 23. Avantageusement, la solidification du matériau de remplissage dans la au moins rainure 231 axiale renforce la solidarisation selon le degré de liberté en rotation autour de l'axe z. Avantageusement, la solidification du matériau de remplissage dans la au moins rainure 231 transverse renforce la solidarisation selon le degré de liberté en translation suivant l'axe z. Avantageusement, la solidification du matériau de remplissage dans la au moins rainure 231 oblique renforce la solidarisation selon les degrés de liberté en translation et en rotation suivant l'axe z. Avantageusement, la solidification du matériau de remplissage dans la au moins une rainure 231 de section non constante renforce la solidarisation selon le degré de liberté en translation et en rotation suivant l'axe z. Selon un mode de réalisation, les rainures 231 permettent en outre l'injection du matériau de remplissage jusque dans la potion interne antérieure 23 renforçant davantage la solidarisation. Selon un mode de réalisation, le matériau de remplissage injecté dans l'évidement cylindrique 122 rempli le au moins un orifice traversant 124, la chambre périphérique 123 et la au moins une rainure 231 de la portion interne antérieure 23, et tout ou partie du volume de la portion interne antérieure 23. Avantageusement, la chambre périphérique 123 permet d'atteindre les rainures 231 indépendamment de l'orientation de l'implant intervertébral 1, et notamment de l'orientation de la portion postérieure 128, dans la fixation pédiculaire 2.

Selon un mode de réalisation, tel qu'illustré sur la Figure 9 A, la partie postérieure de l'implant intravertébral 12 est solidarisée dans la fixation pédiculaire 2 par l'intermédiaire d'un bouchon conique d'expansion 6. Ledit bouchon conique comprend un filetage externe et une extrémité postérieure évasée. Ledit bouchon 6 est inséré au travers de la portion interne postérieure 22, optionnellement après injection de ciment dans la vertèbre par l'intermédiaire de l'orifice traversant 131, et visé dans le taraudage 126 de la portion postérieure 128. Lors du vissage du bouchon conique 6 dans le taraudage 126 de la portion postérieure 128, l'extrémité évasée du bouchon butte contre l'extrémité évasée de la portion postérieure 127 entraînant le déploiement radiale de la portion postérieur, rendu possible grâce aux fentes 125. Avantageusement, le déploiement radial de la portion postérieure 128 dans la portion interne antérieure 23 solidarise la partie postérieure de l'implant intravertébral 12 dans la fixation pédiculaire 2 par appui.

Selon un mode de réalisation, une fois l'implant intravertébral 1 déployé, solidarisé à la fixation pédiculaire 2 et stabilisé, si besoin au moyen de l'injection de ciment ou de substitut osseux spécialement adapté, il est alors possible de solidariser (i.e. connecter) à l'ensemble fixation pédiculaire 2 - implant intravertébral 1 l'élément postérieur 3 par simple vissage et serrage de ce dernier dans la portion interne postérieure 22 de la fixation pédiculaire 2. Dans ce mode de réalisation, l'élément postérieur 3 est au moins partiellement extérieur à la vertèbre 4 et est alors capable de recevoir des éléments postérieurs de fixation tels que des barres ou systèmes de ligaments artificiels ou tout autre moyen de stabilisation de la fracture vertébrale connu de l'homme du métier.

Une méthode de traitement des fractures vertébrales, non revendiquée, comprend:
- l'insertion d'un système d'implant dont la partie postérieure 12 est déjà insérée dans la portion interne antérieure 23 dans une vertèbre 4 et le vissage de la fixation pédiculaire 2 dans le pédicule vertébrale par l'intermédiaire du filetage externe 21 ;
- l'expansion de la partie antérieure 11 de l'implant intravertébral 1, notamment par traction du tube de traction centrale 13 ;
- le positionnement de la partie antérieure 11 de l'implant intravertébral dans le corps vertébral rendu possible par le déplacement de la partie postérieure 12 de l'implant intravertébral 1 en translation et en rotation, selon l'axe principal de la fixation pédiculaire 2, dans la portion interne antérieure 23 de la fixation pédiculaire 2 ; optionnellement, l'injection d'un matériau de remplissage dans le corps vertébral par l'intermédiaire d'un orifice traversant 131 pour stabiliser la partie antérieure de l'implant intravertébral 11 ; et
- optionnellement, la solidarisation de la partie postérieure 12 dans la portion interne antérieure 23.

## Revendications

1. Système d'implant intravertébral expansible comprenant:
un implant intravertébral (1) comprenant :
une partie postérieure (12) , et
une partie antérieure (11) expansible configurée pour placement dans un corps vertébral, et comprenant un premier et un deuxième plateaux, aptes à former respectivement une première et une deuxième surfaces d'appui dans le corps vertébral ; et
une fixation pédiculaire (2) comprenant une portion interne postérieure (22) et une portion interne antérieure (23) définissant une portion creuse de réception de la partie postérieure (12) de implant intravertébral (1) ;
charactérisée en ce que la partie postérieure (12) de l'implant intravertébral (1) peut se déplacer selon au moins un degré de liberté relative à la fixation pédiculaire (2), et en ce que les deux surfaces d'appui étant appelées à s'éloigner l'une dans l'autre suivant un plan d'expansion prédéfini.

2. Système d'implant intravertébral expansible selon la revendication 1, dans lequel la fixation pédiculaire (2) comprend un filetage (21) assurant l'ancrage de ladite fixation pédiculaire dans un pédicule vertébral.

3. Système d'implant intravertébral expansible selon l'une quelconque des revendications 1 à 2, dans lequel la portion interne antérieure (23) comprend au moins une rainure (231) sur sa surface interne.

4. Système d'implant intravertébral expansible selon l'une quelconque des revendications 1 à 3, dans lequel une portion postérieure (128) de la partie postérieure de l'implant intravertébral (12) comprend un évidement cylindrique (122) et au moins un moyen de solidarisation permettant de bloquer en rotation et en translation les mouvements relatifs entre l'implant intervertébral (1) et la fixation pédiculaire (2).

5. Système d'implant intravertébral expansible selon l'une quelconque des revendications 1 à 4, comprenant en outre un élément postérieur (3), partiellement extérieur à la vertèbre et solidaire de la fixation pédiculaire (2).

6. Système d'implant intravertébral expansible selon la revendication 5, dans lequel l'élément postérieur (3) est solidaire de la fixation pédiculaire (2) par l'intermédiaire d'une tige filetée (311) vissée dans un taraudage (221) de la portion interne postérieure (22).

7. Système d'implant intravertébral expansible selon l'une quelconque des revendications 5 ou 6, dans lequel l'élément postérieur (3) comprend une portion postérieure (32) permettant l'assemblage avec des éléments de fixation postérieurs complémentaires tels que des barres ou des ligaments artificiels.

8. Système d'implant intravertébral expansible selon l'une quelconque des revendications 1 à 7, dans lequel la partie postérieure (12) de l'implant intervertébral (1) est coulissante dans la portion interne antérieure (23) et, optionnellement, dans lequel cette liaison coulissante permet l'adaptation de la position de l'implant intravertébral (1) déployé dans le corps vertébral (30) indépendamment de la position de la fixation pédiculaire (2).

9. Système d'implant intravertébral expansible selon l'une quelconque des revendications 1 à 8, dans lequel la partie postérieure (12) comprend un épaulement (121) que sert de butée de guidage pour la fixation pédiculaire (2).

10. Système d'implant intravertébral expansible selon l'une quelconque des revendications 1 à 9, dans lequel la fixation pédiculaire (2) comprend un chanfrein (25) situé a l'extrémité antérieure de la fixation pédiculaire (2) pour faciliter l'insertion de la fixation pédiculaire (2) dans le corps vertébral.

11. Système d'implant intravertébral expansible selon l'une quelconque des revendications 1 à 10, dans lequel la portion interne antérieure (23) ne comprend pas de filetage.

12. Système d'implant intravertébral expansible selon l'une quelconque des revendications 1 à 11, dans lequel la partie postérieure (12) peut coulisser librement dans la portion interne antérieure (23).

13. Système d'implant intravertébral expansible selon l'une quelconque des revendications 1 à 12, dans lequel la fixation pédiculaire (2) comprend au moins une encoche (24) permettant d'entraîner en rotation la fixation pédiculaire (2) a l'aide d'un instrument d'insertion (5).

## Patentansprüche

1. Expandierbares intravertebrales Implantatsystem umfassend:
ein intravertebrales Implantat (1) umfassend:
einen hinteren Teil (12) und
einen expandierbaren vorderen Teil (11), der zum Einbringen in einen Wirbelkörper ausgebildet ist und eine erste und eine zweite Platte umfasst, die jeweils eine erste und eine zweite Auflagefläche in einem Wirbelkörper zu bilden vermögen; und
eine pedikulären Fixation (2) mit einem hinteren Innensegment (22) und einem vorderen Innensegment (23), die ein hohles Segment zur Aufnahme des hinteren Teils (12) des intravertebralen Implantats (1) bilden;
**dadurch gekennzeichnet, dass** der hintere Teil (12) des intravertebralen Implantats (1) in wenigstens einem Freiheitsgrad bezüglich der pedikulären Fixation (2) beweglich ist, und dass die beiden Auflageflächen geeignet sind, sich in einer vordefinierten Expansionsebene voneinander wegzubewegen.

2. Expandierbares intravertebrales Implantatsystem nach Anspruch 1,
wobei die pedikuläre Fixation (2) ein Gewinde (21) aufweist, das die Verankerung der pedikulären Fixation in einem Wirbelpedikel gewährleistet.

3. Expandierbares intravertebrales Implantatsystem nach einem der Ansprüche 1 bis 2,
wobei das vordere Innensegment (23) an seiner Innenseite wenigstens eine Nut (231) aufweist.

4. Expandierbares intravertebrales Implantatsystem nach einem der Ansprüche 1 bis 3,
wobei ein hinterer Abschnitt (128) des hinteren Teils (12) des intravertebralen Implantats eine zylindrische Aussparung (122) und wenigstens ein Verbindungsmittel aufweist, das die Relativbewegungen zwischen dem intravertebralen Implantat (1) und der pedikulären Fixation (2) gegen eine Dreh- und eine Translationsbewegung zu blockieren erlaubt.

5. Expandierbares intravertebrales Implantatsystem nach einem der Ansprüche 1 bis 4,
ferner umfassend ein hinteres Elements (3), das zum Teil außerhalb des Wirbels liegt und mit der pedikulären Fixation (2) fest verbunden ist.

6. Expandierbares intravertebrales Implantatsystem nach Anspruch 5,
wobei das hintere Element (3) mit der pedikulären Fixation (2) mittels eines Gewindestifts (311) fest verbunden ist, der in ein Gewinde (221) des hinteren Innensegments (22) geschraubt ist.

7. Expandierbares intravertebrales Implantatsystem nach einem der Ansprüche 5 oder 6,
wobei das hintere Element (3) ein hinteres Segment (32) umfasst, das eine Verbindung mit komplementären hinteren Fixationselementen wie Stangen oder künstlichen Bändern erlaubt.

8. Expandierbares intravertebrales Implantatsystem nach einem der Ansprüche 1 bis 7,
wobei der hintere Teil (12) des intravertebralen Implantats (1) in dem vorderen Innensegment (23) verschiebbar ist und optional diese verschiebbare Verbindung die Anpassung der Lage des in dem Wirbelkörper (30) eingebrachten intravertebralen Implantats (1) unabhängig von der Lage der pedikulären Fixation (2) ermöglicht.

9. Expandierbares intravertebrales Implantatsystem nach einem der Ansprüche 1 bis 8,
wobei der hintere Teil (12) einen Absatz (121) aufweist, der der pedikulären Fixation (2) als Führungsanschlag dient.

10. Expandierbares intravertebrales Implantatsystem nach einem der Ansprüche 1 bis 9,
wobei die pedikuläre Fixation (2) eine am vorderen Ende der pedikulären Fixation (2) angeordnete Abfasung (25) aufweist, um das Einsetzen der pedikulären Fixation (2) in den Wirbelkörper zu erleichtern.

11. Expandierbares intravertebrales Implantatsystem nach einem der Ansprüche 1 bis 10,
wobei das vordere Innensegment (23) kein Gewinde aufweist.

12. Expandierbares intravertebrales Implantatsystem nach einem der Ansprüche 1 bis 11,
wobei der hintere Teil (12) in dem vorderen Innensegment (23) frei gleiten kann.

13. Expandierbares intravertebrales Implantatsystem nach einem der Ansprüche 1 bis 12,
wobei die pedikuläre Fixation (2) wenigstens eine Einkerbung (24) aufweist, durch die die pedikuläre Fixation (2) mit Hilfe eines Einsetzinstruments (5) in Drehung versetzt werden kann.

## Claims

1. An expandable intravertebral implant system comprising:
an intravertebral implant (1) comprising:
a posterior part (12), and
an expandable anterior part (11) configured for placement in a vertebral body, and comprising a first plate and a second plate, able to form respectively a first and a second bearing surface in the vertebral body; and
a pedicle fixation (2) comprising an inner posterior portion (22) and an inner anterior portion (23) defining a hollow portion receiving the posterior part (12) of the intravertebral implant (1);
**characterized in that** the posterior part (12) of the intravertebral implant (1) is movable according to at least one degree of freedom relative to the pedicle fixation (2), and **in that** the two bearing surfaces are intended to move away from each other according to a predefined expansion plane.

2. The expandable intravertebral implant system according to claim 1, wherein the pedicle fixation (2) comprises a thread (21) providing anchorage of said pedicle fixation into a vertebral pedicle.

3. The expandable intravertebral implant system according to any of the claims 1 to 2, wherein the inner anterior portion (23) comprises at least one groove (231) on its inner surface.

4. The expandable intravertebral implant system according to any of the claims 1 to 3, wherein a posterior portion (128) of the posterior part (12) of the intravertebral implant comprises a cylindrical recess (122) and at least a fastening means enabling to rotationally and translationally lock the relative movements between the intravertebral implant (1) and the pedicle fixation (2).

5. The expandable intravertebral implant system according to any of the claims 1 to 4, further comprising a posterior element (3), partially outside of the vertebra and integral with the pedicle fixation (2).

6. The expandable intravertebral implant system according to claim 5, wherein the posterior element (3) is integral with the pedicle fixation (2) by means of a threaded pin (311) screwed in a threaded bore (221) of the inner posterior portion (22).

7. The expandable intravertebral implant system according to any of the claims 5 or 6, wherein the posterior element (3) comprises a posterior portion (32) enabling assembly with complementary posterior fixation elements such as bars or artificial ligaments.

8. The expandable intravertebral implant system according to any of the claims 1 to 7, wherein the posterior part (12) of the intravertebral implant (1) is slidable in the inner anterior portion (23) and, optionally, wherein this slidable connection enables adaptation of the position of the intravertebral implant (1) deployed in the vertebral body (30) regardless of the position of the pedicle fixation (2).

9. The expandable intravertebral implant system according to any of the claims 1 to 8, wherein the posterior part (12) comprises a shoulder (121) which acts as a guiding abutment for the pedicle fixation (2).

10. The expandable intravertebral implant system according to any of the claims 1 to 9, wherein the pedicle fixation (2) comprises a bevel (25) located at the anterior end of the pedicle fixation (2) to facilitate insertion of the pedicle fixation (2) into the vertebral body.

11. The expandable intravertebral implant system according to any of the claims 1 to 10, wherein the inner anterior portion (23) has no thread.

12. The expandable intravertebral implant system according to any of the claims 1 to 11, wherein the posterior part (12) is freely slideable in the inner anterior portion (23).

13. The expandable intravertebral implant system according to any of the claims 1 to 12, wherein the pedicle fixation (2) comprises at least one notch (24) enabling to rotatively drive the pedicle fixation (2) by means of an insertion instrument (5).
